# EUROPEAN PATENT APPLICATION

(11) **EP 2 452 620 A1**
(43) Date of publication of application: **16.05.2012**
(21) Application number: 11002714.1
(22) Date of filing: 01.04.2011
(51) Int. Cl.: A61B 5/0215, A61B 5/026

(54) **An angiographic catheter for the measurement of the transmission speed of the aortic pressure wave**

(30) Priority: 10.11.2010 IT MI20102080
(71) Applicant: FLAG VASCULAR SRL, 20900 Monza (MB) (IT)
(72) Inventor: Scalise, Filippo, 20900 Monza (MB) (IT)

(57) **Abstract**

An angiographic catheter (10) for the measurement of the transmission speed of the arterial pressure wave inside a blood vessel, as for example an artery and in particular an artery aorta, which comprises a first tubular element (20) provided with a first lumen (22) and a second tubular element (30) provided with a second lumen (32), the first tubular element (20) and the second tubular element (30) being realized from a flexible polymeric material and said second tubular element (30) further being partially inserted and sliding inside the first lumen (22) of the first tubular element (20), the first lumen (22) comprising a first proximal aperture (23), which can be connected with a first pressure transducer capable to detect a first arterial pressure wave, and furthermore said second lumen (32) comprises a second proximal aperture (33), which can be connected with a second pressure transducer capable to directly detect a second arterial pressure wave, the second tubular element (30) comprising a graduated scale (37) which is realized on an external surface of said second tubular element (30) on a proximal portion (36) of the same, said graduated scale (37) partially externally protruding with respect to said first tubular element (20) and so permitting to directly read anytime the real exact distance between said second distal aperture (35) and said first distal aperture (25) along said blood vessel.

## Description

The present invention refers to an angiographic catheter for the invasive measurement of the pressure wave at the same time in two points inside a blood vessel as in particular an artery preferably an aorta.

In particular the present invention refers to an angiographic catheter able to permit the invasive measurement of the arterial pressure wave, the velocity of transmission of the arterial pressure wave inside one artery preferably an aorta of a person.

Through the measurement of the pressure wave, it is then possible to determine a set of parameters, among which the transmission velocity of the aortal pressure wave which is related to the aortic rigidity, a very important parameter for evaluating the operation of a cardio-vascular system.

Today for determining the shape of the arterial pressure wave and of the velocity of transmission of the pressure wave inside an artery, noninvasive systems and methods are used.

A first disadvantage is in that the noninvasive measurement systems and methods do not permit the direct measurement of the shape of the arterial pressure wave.

The arterial pressure wave is the result of a anterograde wave superimposed on a reflected retrograde wave.

Therefore the complexity of this phenomenon is difficult to measure by means of an indirect measurement.

Another disadvantage of the indirect measurement systems and methods is that they do not permit the precise detection of the exact morphology of the arterial pressure wave.

Furthermore these non-invasive measurement systems and methods permit an estimation of the propagation speed of the arterial pressure wave, always by means o fan indirect measurement obtained through two measurements in two peripheral points of the peripheral pressure waves from which it is possible to derive the arterial pressure waves in the two corresponding points.

Through a comparison of the two arterial pressure waves derived it is possible to determine the propagation time of the arterial pressure wave.

The propagation speed of the arterial pressure wave is determined by dividing the distance between two points by the propagation time of the arterial pressure wave, for then determining the aortic rigidity.

Another disadvantage is that the pressure wave propagates between the two measurement points along a path which does not correspond with the linear distance between the two points, and this determines an error in the propagation speed of the arterial pressure wave and consequently also in the calculation of the aortic rigidity.

Purpose of the present invention is to realize an angiographic catheter for the measurement of the transmission speed of the arterial pressure wave, which permits a precise measurement of the propagation speed of the arterial pressure wave and which permits a very precise calculation of the arterial rigidity.

Another purpose is to realize an angiographic catheter for the measurement of the transmission speed of the arterial pressure wave which is easy to realize and at the same time to be inserted and positioned inside an arterial vessel.

Still another purpose is to have a device for measuring the rigidity of a blood vessel,, which permits a continuous invasive measurement of the blood pressure wave in two points and permits to precisely determine a set of parameters among which the vascular rigidity.

Further purpose is to have an angiographic catheter for the measurement of the transmission speed of the arterial pressure wave, which is economically advantageous and simple to realize.

These purposes according to the present invention are reached by realizing an angiographic catheter for the measurement of the transmission speed of the arterial pressure wave, as explained in claim 1.

Further characteristics of the invention are highlighted by the subsequent claims.

The characteristics and advantages of an angiographic catheter for the measurement of the transmission speed of the arterial pressure wave according to the present invention, will be more evident from the following exemplary and non limitative description referring to the annexed schematic drawings, in which:

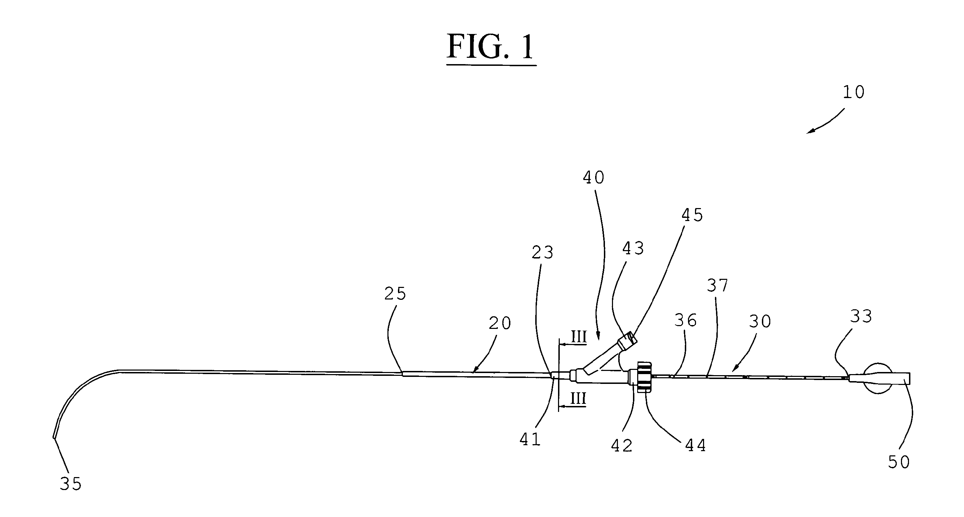

figure 1 is a raised lateral left view of a preferred embodiment of an angiographic catheter for the measurement of the transmission speed of an arterial pressure wave according to the present invention,

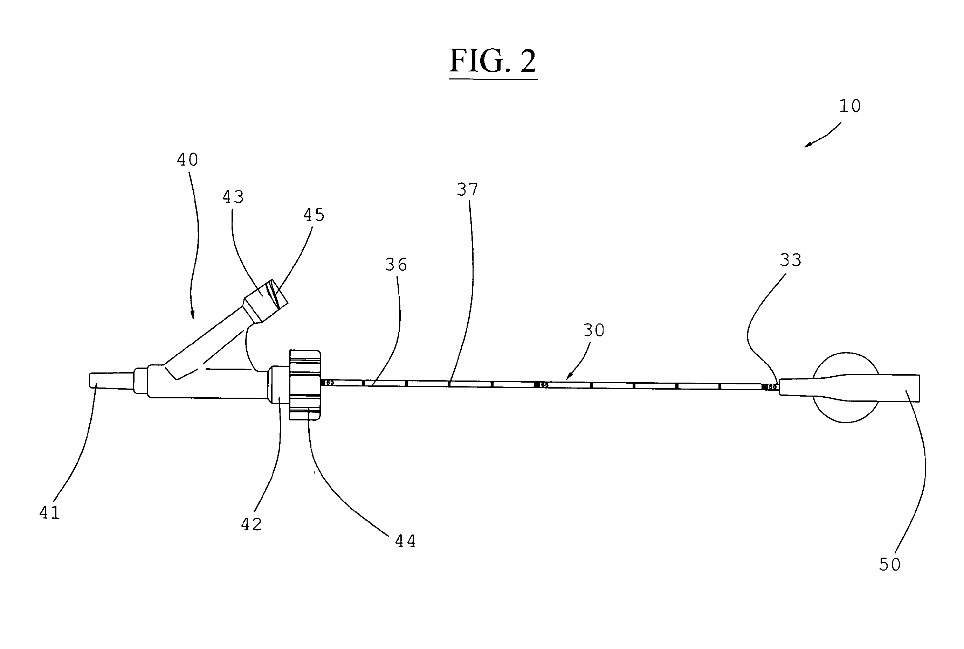

figure 2 is partially sectioned raised lateral left view of a detail of figure 1;

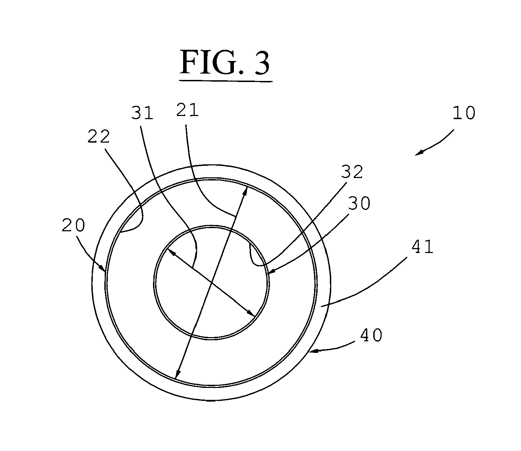

figure 3 is a raised frontal view of a preferred embodiment of a section of the angiographic catheter of figure 1 section according to the line III-III.

With reference to the figures, an angiographic catheter 10 is shown, for the measurement of the transmission speed of the arterial pressure wave inside a blood vessel, as for example an artery and in particular an aorta, which comprises a first tubular element 20 provided with a first lumen 22 and a second tubular element 30 provided with a second lumen 32, said first tubular element 20 and said second tubular element 30 being realized of a flexible polymeric material and furthermore said second tubular element 30 is partially inserted and can slide inside said lumen 22 of said first tubular element 20.

Said angiographic catheter 10 can be partially inserted into said blood vessel, which is in particular an artery and in particular an artery aorta, furthermore said first tubular element 20 can be completely inserted into said blood vessel which is in particular an artery and in particular an artery aorta, furthermore said first tubular element 20 can be partially inserted into said blood vessel.

According to the present invention said first lumen 22 comprises a first proximal aperture 23, which is connectable with a first pressure transducer able to directly detect a second electrical signal directly proportional to a blood pressure wave, in particular an arterial one, and further it comprises a first distal portion provided with a first distal aperture 25 positioned at a second free extremity of said distal portion.

Furthermore said second lumen 32 comprises a second proximal aperture 33, which is connectable with a second pressure transducer able to directly detect a first electrical signal directly proportional to a blood pressure wave, in particular an arterial one, and preferably an aortic one, and further it comprises a second distal portion provided with a second distal aperture 35 positioned at a second free extremity of said second distal portion.

The second tubular element 30 comprises a graduated scale 37, which in particular is a metric graduated scale or a graduated scale of anglo-saxon type, which is realized on an outer surface of said second tubular element 30 on a proximal portion 36 of the same, in particular near said second proximal aperture 33, such graduated scale 37 partially protruding ad the outside of said first tubular element 20, in particular also protruding at the outside of said blood vessel of a patient, and so it permits to directly read anytime the exact real distance between said second distal aperture 35 and said first distal aperture 25 along said blood vessel, which in particular is an artery and in particular an artery aorta.

Advantageously in this way it is possible to easily determine a vascular rigidity in particular an arterial rigidity like for example an aortic rigidity.

In particular said graduated scale 37 comprises a graduated portion externally protruding with respect to said first proximal extremity 23 of said first tubular element 20, and which furthermore directly indicates said real distance between said second distal aperture 35 and said first distal aperture 25 in particular along said blood vessel, in particular an arterial one, so permitting anytime a very precise measurement of the same, in an extremely easy way as it is simply possible to make a direct reading of the portion of graduated scale which is externally visible from said first tubular element 20 mainly when in particular said first tubular element 20 is partially inserted into said blood vessel of a patient.

Advantageously this permits a very precise measurement of the aortic rigidity as it is possible to know anytime the real distance between said second distal aperture 35 and said first distal aperture 25, and at the same time it advantageously permits to vary said distance on the basis of the anatomy of the patient, so avoiding the need of realizing more angiographic catheters 10 with different predetermined distances between said second distal aperture 35 and said first distal aperture 25.

Advantageously, said catheter permits to perform at the same time two measurements of the blood pressure wave in particular of the arterial one in two points placed at a predetermined distance along a vascular duct which preferably is an artery, said predetermined distance being immediately indicated on the portion of said graduated scale which protrudes at the outside of said first tubular element 20, consequently permitting to precisely and simply avoid an arterial rigidity in particular an aortic rigidity.

As a matter of fact said blood vessel, in particular an artery and preferably an aorta, typically extends along a non linear path, so that typically the distance between two points along said path does not correspond to the linear distance between the two points themselves, and so it would determine an error in the calculation of the aortic rigidity, as in the case of a non-invasive measurement system, by determining the errors when obtaining the same.

Advantageously it is in this way possible to make many measurements of the vascular rigidity of vascular portions of various known lengths in order to permit to highlight a plurality of pathologies, and their position, which alter the arterial wall and its elasticity.

Preferably said first tubular element 20 and said second tubular element 30 are realized with a polymeric material in particular based on nylon and/or its similar ones.

Said polymeric material is preferably a polymeric material based on nylon and/or its similar ones.

Advantageously this permits to have an angiographic catheter 10 which is economical and easy to realize.

Preferably said first tubular element 20 and said second tubular element 30 are realized from a polymeric material comprising a nylon-based polymer and/or its similar ones mixed with an inert radio-opaque additive, as in particular barium sulfate, which is radio-opaque to the X-rays.

In particular said radio-opaque inert additive is present in said polymeric material with a percentage comprised between 10 % and 55 % and in particular comprised between 22% and 40 %.

Advantageously this permits to have an angiographic catheter 10 which is economical and easy to realize and which at the same time is easy to be inserted and positioned as it anytime permits to visualize the angiographic catheter inserted in a patient simply by irradiating the patient with the X-rays.

In particular said second tubular element 30 has a length greater than said first tubular element 20, so that said second distal aperture 35 permits the measurement of a central blood pressure wave in particular an arterial one and preferably an aortic one, whereas said first distal aperture 25 permits the measurement of a peripheral blood pressure wave in particular an arterial one.

Preferably said first distal aperture 25 and said second distal aperture 35 are mutually spaced at a predetermined distance preferably being greater than 40 cm and in particular greater than 69 cm, or order to permit an adaptability to different heights and body sizes of different patients.

Said second lumen 32 comprises an internal diameter 31 which is preferably greater than 0,6 mm and in particular greater than 1 mm, still more particularly greater than or equal to 1,2 mm.

Said first lumen 22 comprises an internal diameter 21 which is preferably comprised between 1,1 mm and 1,9 mm and in particular is comprised between 1,6 and 1,8 mm.

Advantageously from the experimental point of view this mainly permits to avoid an attenuation of the two aortic pressure signals which would determine severe errors of measurement of the transmission speed of the pressure wave and of determination of the aortic rigidity.

Furthermore the attenuation of the two aortic pressure signals measured by means of said first lumen 22 and by means of said second lumen 32 would not permit to determine a set of other parameters very important for evaluating the conditions of said artery and so even the conditions of a cardio-vascular system of a patient.

Advantageously at the same time this permits to have an angiographic catheter 10 of reduced dimensions, in order not to cause damages to a patient, by reducing at the minimum the disease for the patient himself.

Preferably said angiographic catheter 10 comprises a substantially Y-shaped valve 40 which has a first distal extremity 41, a second proximal extremity 42 and a third proximal extremity 43.

In particular said first distal extremity 41 is connected with said first proximal extremity 23 of said first tubular element 20, and preferably it is made integral with the same in order to make them coaxial.

Said first distal extremity 41 and said second proximal extremity 42 are substantially aligned and coaxial in order to permit the insertion into the same of a second tubular element 30 in order to facilitate the sliding of said second tubular element 30 into said valve 40 and into said first tubular element 20, so avoiding a possible contact and a possible internal friction of said second tubular element 30 inside said valve 40.

Said valve 40 further comprises polymeric tightening means and a closure tap which are bound to said second proximal extremity 42 in order to permit to said second tubular element 30 to slide within said valve 40 relatively with respect to said first tubular element 20, at the same time preventing a spill of blood from said valve 40.

Said third proximal extremity 43 can be connected with said second pressure transducer which is able to detect said second electric signal directly proportional to a blood pressure wave, in particular arterial, in a second peripheral point with respect to said first point along said vascular in particular arterial duct.

Furthermore preferably at said third proximal extremity 43 a threaded connection 45 of the luer lock type is realized.

Preferably said second polymeric tubular element 30 comprises at least 3 tubular portions mutually adjacent and realized in a single piece and mutually coaxial, a first proximal tubular portion, a second central tubular portion and a third distal tubular portion, said third distal tubular portion being less rigid than said second central tubular portion and said first proximal tubular portion, and further said second central tubular portion being less rigid than said first proximal tubular portion.

In other words said first proximal tubular portion, said second central tubular portion and said third distal tubular portion have a decreasing rigidity the one with respect to the adjacent one, going towards said second distal aperture 35.

Advantageously in this way a decreasing rigidity is obtained and therefore an increasing flexibility, going from said second proximal aperture 33 towards said second distal aperture 35 so obtaining an angiographic catheter 10 simple to realize and extremely handy and simple to use.

Preferably said third distal portion of said second tubular element 30 is curved at said second distal aperture 35 in order to facilitate the insertion of said angiographic catheter 10 in a blood vessel, in particular avoiding the contact of said second distal aperture 35 with the internal walls of said blood vessel in particular arterial.

Preferably said angiographic catheter 10 further comprises a first connection duct which can be screwed with said third proximal extremity 43 of said valve 40 by means of a threaded connection 45 fixed or made integral with the same, in particular of the "luer lock" type, and furthermore said first connecting duct can be connected with a second extremity of said second pressure transducer which continuously detects said second electric signal directly proportional to said blood pressure wave, in particular arterial.

Preferably said angiographic catheter 10 further comprises a second connecting duct which can be screwed with said second proximal extremity 33 of said second tubular element 30 by means of a threaded connection 50 fixed or made integral with the same, in particular of the "luer lock" type, and furthermore said second connecting duct can be connected with said second extremity of said first pressure transducer which continuously detects said first electric signal directly proportional to said blood pressure wave, in particular arterial and preferably aortic.

According to a further aspect of the present invention a device is provided for the measurement of the rigidity of a blood vessel, in particular arterial and preferably aortic, which comprises an angiographic catheter 10 according to any of the described embodiments and constructive variations, and further it comprises a first pressure transducer and a second pressure transducer, and furthermore it comprises electronic means for data acquisition and processing, which can be connected with said first and second pressure transducer in order to continuously and at the same time detect a first and second pressure wave in particular arterial, and for calculating a set of parameters regarding the state and function of a blood vascular system and of a cardiovascular system.

Said electronic acquisition data in particular comprise filtering and amplifying means, analogue-digital conversion means.

Preferably said measurement device further comprises one display and one computer connected with said electronic data acquisition means and further it comprises at least one mass memory device or at least one static memory.

Preferably said first pressure transducer is connected with said first proximal extremity 23 of said first tubular element 20, and furthermore said second pressure transducer is connected with said second proximal extremity 33 of said second tubular element 30.

In particular according to a preferred embodiment said first pressure transducer is a first micro pressure transducer which is connected with said first distal extremity 25 of said first tubular element 20, and furthermore said second pressure transducer is a second micro pressure transducer which is connected with said second distal extremity 35 of said second tubular element 30 in order to make much more precise the continuous measurement of said first and second pressure wave in particular arterial, at the same time in two distinct points of an arterial vessel.

So it has been seen that an angiographic catheter for the measurement of the arterial pressure wave inside a blood vessel according to the present invention realizes the previously highlighted purposes.

The so conceived angiographic catheter for the measurement of the pressure wave inside an artery of the present invention, can be subject to many modifications and variations, all within the same inventive concept.

Furthermore, in practice the used materials, and also their dimensions and components, can be of any kind according to the technical needs.

## Claims

1. An angiographic catheter (10) for the measurement of the transmission speed of the arterial pressure wave inside a blood vessel, which comprises a first tubular element (20) provide with a first lumen (22) and a second tubular element (30) provide with a second lumen (32), said first tubular element (20) and said second tubular element (30) being realized from a flexible polymeric material and said second tubular element (30) further being partially inserted and sliding inside said first lumen (22) of said first tubular element (20), and said first tubular element (20) can be partially inserted internally inside said blood vessel, said angiographic catheter (10) being **characterized in that** said first lumen (22) comprises a first proximal aperture (23), which can be connected with a second pressure transducer capable to directly detect a second electric signal directly proportional to a second blood pressure wave and furthermore it comprises a first distal portion provided with a first distal aperture (25) positioned at a second free extremity of said first distal portion, and furthermore said second lumen (32) comprises a second proximal aperture (33), which can be connected with a second pressure transducer and capable to directly detect a first electric signal directly proportional to a second blood pressure wave and furthermore it comprises a second distal portion provided with a second distal aperture (35) positioned at a second free extremity of said second distal portion, said second tubular element (30) comprising a graduated scale (37) which is realized on an external surface of said second tubular element (30) on a proximal portion (36) of the same, in particular near said second proximal aperture (33), said graduated scale (37) partially externally protruding with respect to said first tubular element (20) and so permitting to directly read anytime the real exact distance between said second distal aperture (35) and said first distal aperture (25) along said blood vessel.

2. The angiographic catheter (10) according to claim 1, **characterized in that** said graduated scale (37) comprises a graduated portion which externally protrudes with respect to said first proximal extremity (23) of the first tubular element (20), and which further directly indicates said real distance between said second distal aperture (35) and said first distal aperture (25) in particular along said blood vessel, in particular arterial, so permitting anytime a very precise measurement of the same, in an extremely simple manner as it is simply possible a direct reading of the portion of graduated scale externally visible from said first tubular element (20).

3. The angiographic catheter (10) according to claim 1 or 2, **characterized in that** said first tubular element (20) and said second tubular element (30) are realized from a polymeric material comprising a nylon-based polymer and/or its similar blended with an inert radio-opaque additive which is radio-opaque to the X-rays.

4. The angiographic catheter (10) according to claim 3, **characterized in that** said radio-opaque inert additive is present in said polymeric material with a percentage comprised between 10 % and 55 % .

5. The angiographic catheter (10) according to any of claims 1 to 4, **characterized in that** said second lumen (32) comprise an internal diameter (31) which is preferably greater than 0,6 mm, said first lumen (22) further comprising an internal diameter (21) which is preferably comprised between 1,1 mm and 1,9 mm.

6. The angiographic catheter (10) according to any of claims 1 to 4, **characterized in that** it comprise a substantially Y-shaped valve (40) which presents a first distal extremity (41), a second proximal extremity (42) and a third proximal extremity (43), said first distal extremity (41) being connected with said first proximal extremity (23) of said first tubular element (20), furthermore said first distal extremity (41) and said second proximal extremity (42) being substantially aligned and coaxial in order to permit the insertion into the same of this second tubular element (30) in order to facilitate the sliding of said second tubular element (30) inside said valve (40) and inside said first tubular element (20).

7. The angiographic catheter (10) according to claim 6, **characterized in that** said valve (40) comprises polymeric tightening means and a closure tap which are bound to said second proximal extremity (42) in order to permit the sliding of said second tubular element (30) inside said valve (40) relatively with respect to said first tubular element (20), at the same time preventing a spill of blood from said valve (40).

8. The angiographic catheter (10) according to claim 6 or 7, **characterized in that** at said third proximal extremity (43) a threaded connection is realized of the luer lock type.

9. The angiographic catheter (10) according to any of claims 1 to 8, **characterized in that** said second tubular element (30) comprise at least 3 tubular portions mutually adjacent and realized in a single piece and mutually coaxial, a first tubular proximal portion, a second tubular proximal portion and a third tubular distal portion, said third tubular distal portion being less rigid than said second central tubular portion and of said first tubular proximal portion, and furthermore said second central tubular portion being less rigid than said first tubular proximal portion.

10. The angiographic catheter (10) according to claim 9, **characterized in that** said third distal portion of said second tubular element (30) is curved at said second distal aperture (35) in order to facilitate the insertion of said angiographic catheter (10) inside a blood vessel, so in particular avoiding the contact of said second distal aperture (35) with the internal walls of said blood vessel and in particular arterial.

11. The angiographic catheter (10) according to any of claims 1 to 10, **characterized in that** it comprises a first connecting duct which can be screwed upon said third proximal extremity (43) of said valve (40) by means of a threaded connection (45) fixed or made integral with the same and furthermore said first connecting duct can be connected at a second extremity with said second pressure transducer which continuously detects said second electric signal directly proportional to said blood pressure wave, in particular arterial, and furthermore said angiographic catheter (10) comprises a second connecting duct which can be screwed upon said second proximal aperture (33) of said second tubular element (30) by means of a threaded connection (50) fixed or made integral with the same and furthermore said second connecting duct can be connected at a second extremity with said first pressure transducer which continuously detects said first electric signal directly proportional to said blood pressure wave, in particular arterial and preferably aortic.

12. A measurement device of the rigidity of a blood vessel, comprising an angiographic catheter (10) according to an y of claims 1 to 11, further comprising a first pressure transducer and a second pressure transducer, and further comprising electronic means for data acquisition and processing, which can be connected with said first and second pressure transducer in order to continuously and at the same time detect a first and a second pressure wave in particular arterial and preferably aortic and for calculating a set of parameters correlated with the state and function of a blood vascular system and of a cardiovascular system, said electronic means of data acquisition in particular comprising filtering and amplifying means and analogue-digital conversion means.

13. The measurement device of the rigidity of a blood vessel according to claim 12, **characterized in that** is comprises a computer provided with a video which is connected with said electronic means for data acquisition and further comprising at least a mass memory device or at least a static memory.
